## Europäisches Patentamt

## European Patent Office

## Office européen des brevets

(19)

(11) Veröffentlichungsnummer: **0 245 584**
**B1**

(12)

# EUROPÄISCHE PATENTSCHRIFT

(45) Veröffentlichungstag der Patentschrift:
23.05.90

(51) Int. Cl.⁵: **A61B 6/00**

(21) Anmeldenummer: 87101523.6

(22) Anmeldetag: 05.02.87

(54) Röntgenuntersuchungsgerät mit einer ein Röntgensystem tragenden Säule.

(30) Priorität: 12.05.86 DE 8612867 U

(43) Veröffentlichungstag der Anmeldung:
19.11.87 Patentblatt 87/47

(45) Bekanntmachung des Hinweises auf die Patenterteilung:
23.05.90 Patentblatt 90/21

(84) Benannte Vertragsstaaten:
DE FR

(56) Entgegenhaltungen:
FR-A- 1 020 382
US-A- 2 822 477
US-A- 2 835 520

(73) Patentinhaber: Siemens Aktiengesellschaft,
Wittelsbacherplatz 2, D-8000 München 2(DE)

(72) Erfinder: Bock, Christian Dipl.-Ing (FH), Siedlung 9a,
D-8525 Uttenreuth(DE)
Erfinder: Kaul, Karlheinz Dipl.-Ing (FH), Ruhsteinweg 4,
D-8525 Uttenreuth(DE)

## Beschreibung

Röntgenuntersuchungsgerät mit einer ein Röntgensystem tragenden Säule.

Die Erfindung betrifft ein Röntgenuntersuchungsgerät gemäß dem ersten Teil des Patentanspruches.

Bei einem Röntgenuntersuchungsgerät dieser Art kann als Röntgensystem ein C-Bogen mit einem Röntgenstrahler und einem Strahlenempfänger an seinen Enden vorgesehen sein. Ein Gerät dieser Art kann für die Angiokardiographie und die interventionelle Technik verwendet werden. Der zu untersuchende Bereich des Patienten wird dabei durch Einstellung des C-Bogens längs seiner Umfangsrichtung, durch Verschwenken des C-Bogens und durch Vefahren der Säule gewählt.

Durch die FR-A 1 020 382 und die US-A 2 822 477 sind Säulen für die Führung eines ein Röntgensystem tragenden Wagens bekannt, die als Kastenprofil ausgebildet sind. Zur Führung des Wagens sind an diesem Kastenprofil zusätzlich Führungsschienen befestigt, in denen der Wagen mit Rollen läuft. Der Herstellungsaufwand, der durch diese zusätzlichen Führungsschienen bedingt ist, ist daher erheblich.

Der Erfindung liegt die Aufgabe zugrunde, ein Röntgenuntersuchungsgerät der eingangs genannten Art gegenüber dem Stand der Technik zu vereinfachen.

Diese Aufgabe ist erfindungsgemäß gelöst durch den zweiten Teil des Patentanspruches. Bei dem erfindungsgemäßen Röntgenuntersuchungsgerät ist ein das Röntgensystem tragender Wagen auf der Säule in Längsrichtung einstellbar, wobei besondere Schienen für diesen Wagen nicht vorgesehen sind. Die Schienen sind von der als Hohlprofil ausgebildeten Säule selbst, und zwar von deren Wandungen gebildet. Die Säule kann dabei zweckmäßigerweise aus Leichtmetall im Strangpreßverfahren hergestellt werden.

Die Erfindung ist nachfolgend anhand eines in der Zeichnung dargestellten Ausführungsbeispieles näher erläutert.
·Es zeigen:

Fig. 1 ein Röntgenuntersuchungsgerät nach der Erfindung, und
Fig. 2 eine Einzelheit des Röntgenuntersuchungsgerätes gemäß Figur 1.

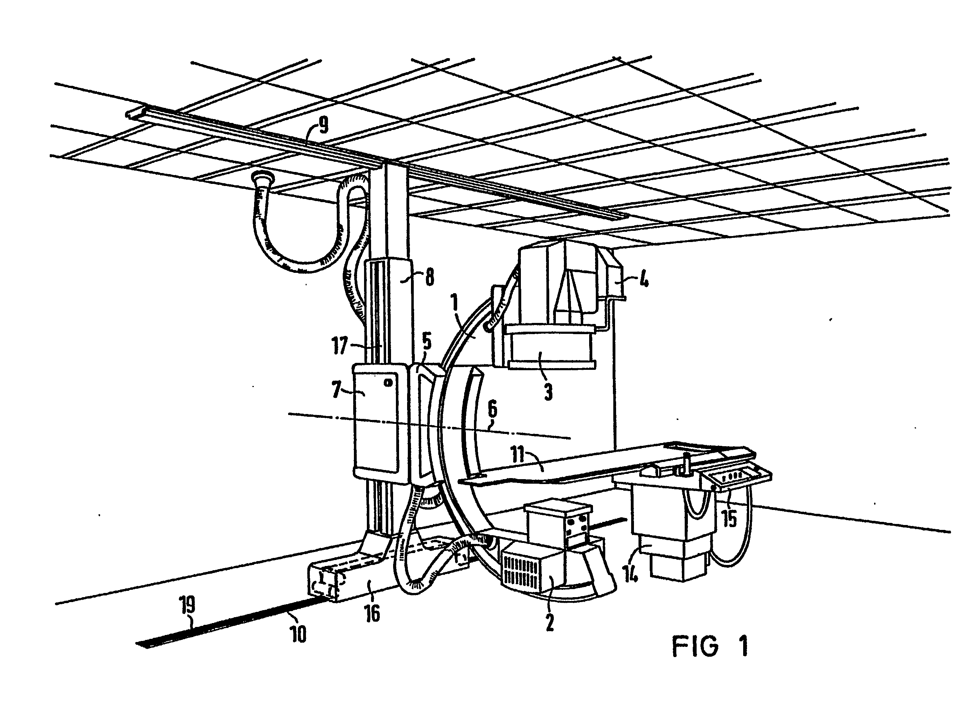

In der Figur 1 ist ein C-Bogen 1 dargestellt, der an seinen Enden einen Röntgenstrahler 2 und einen Röntgenbildverstärker 3 mit nachgeschalteter Einzelbildkamera 4 sowie nicht sichtbarer Fernsehkamera trägt. Der C-Bogen 1 ist längs seines Umfanges an einem Halter 5 verstellbar gelagert, welcher um eine horizontale Achse 6 schwenkbar mit einem Wagen 7 verbunden ist. Der Wagen 7 ist an einer vertikalen Säule 8 längsverschiebbar gelagert, welche in einer Deckenschiene 9 und einer Bodenschiene 10, welche parallel zueinander und zur Längsrichtung eines Patientenlagerungstisches 11 verlaufen, der verschiebbar ist. Dieser C-Bogen 1 umgreift den Patientenlagerungstisch 11 quer. Der Patientenlagerungstisch 11 ist auf einem Sockel 14 höhenverstellbar gelagert, an dem ein Bedienpult 15 befestigt ist. Er ist gegenüber dem Sockel 14 in Längs- und Querrichtung verstellbar (schwimmende Lagerung), wobei jedoch der Stellweg in Längsrichtung relativ klein ist (Größenordnung 40 cm).

Die Wahl des untersuchten Bereiches erfolgt im wesentlichen durch Einstellung des Röntgenstrahlers 2 und des Röntgenbildverstärkers 3, also nicht durch Verstellung des Patientenlagerungstisches 11. Die Einstellung kann dabei in folgender Weise erfolgen:

Zunächst wird die Säule 8 motorisch ganz nach links verfahren. In der so erreichten Stellung wird der C-Bogen 1 in die jeweilige Untersuchungslage gebracht (Bildverstärker 3 oberhalb oder unterhalb des Patientenlagerungstisches 11 oder seitlich von diesem). Anschließend wird die Säule 8 so weit zurückgefahren, bis der Röntgenstrahler 2 und der Röntgenbildverstärker 3 unter bzw. über dem zu durchstrahlenden Bereich des Patienten oder seitlich von diesem liegen. Die jeweilige Höhenlage des Isozentrums wird dabei über den Wagen 7 eingestellt. Schließlich kann die Strahlenrichtung auch noch durch Schwenken des C-Bogens 1 um die Achse 6 variiert werden.

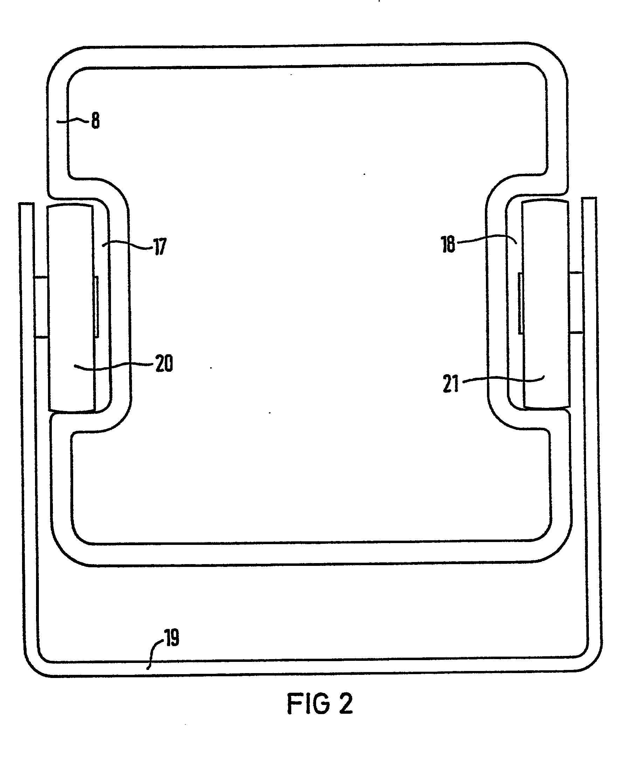

Die Figur 2 zeigt, daß die Säule 8 aus einem geschlossenen Kastenprofil besteht, das an zwei parallelen Längsseiten einander gegenüberliegend je eine U-förmige, in Längsrichtung verlaufende Vertiefung 17, 18 aufweist. Die Vertiefungen 17, 18 dienen als Schienen für den Wagen 7. Vom Wagen 7 ist ein bügelförmiger Teil 19 dargestellt, an dem Räder 20, 21 drehbar gelagert sind, die in den Vertiefungen 17, 18 in Längsrichtung der Säule 8 abrollen.

## Patentansprüche

1. Röntgenuntersuchungsgerät mit einer ein Röntgensystem (2, 3, 4) tragenden Säule (8), auf der ein Wagen (7) längsverschiebbar gelagert ist, der das Röntgensystem (2, 3, 4) trägt, wobei die Säule (8) im Querschnitt ein Kastenprofil aufweist und an zwei parallelen Längsseiten einander gegenüberliegend je eine U-förmige, in Längsrichtung verlaufende Führungsschiene für den Wagen (7) aufweist, dadurch gekennzeichnet, daß die Säule (8) im Querschnitt ein geschlossenes Kastenprofil ist und die Führungsschienen an zwei parallelen Wandungen der Säule (8) als nach außen weisende Vertiefungen (17, 18) ausgebildet sind, die von den Wandungen des Kastenprofils selbst gebildet sind.

## Claims

1. An x-ray examination apparatus with a column (8) carrying an x-ray system (2, 3, 4), on which a truck (7) is mounted in a longitudinally displaceable manner, which carries the x-ray system (2, 3, 4), wherein the column (8) has a box profile in cross-section and has, on two parallel longitudinal sides, opposite each other, a respective U-shaped guide rail for the truck (7), extending in longitudinal direction, characterised in that the column (8) is a closed box profile in cross-section and the guide rails are constructed on two parallel walls of the column (8) as outwardly directed indentations (17, 18) which are formed by the walls of the box profile itself.

**Revendications**

1. Appareil de radiodiagnostic comportant une colonne (8), qui porte un système radiologique (2, 3, 4) et sur laquelle un chariot (7), qui porte le système radiologique (2, 3, 4), est monté de manière à être déplaçable longitudinalement, la colonne (8) possédant un profil en forme de boîte en coupe transversale et comportant, pour le chariot (7), des rails de guidage respectifs en forme de U, qui s'étendent dans la direction longitudinale et qui sont disposés à l'opposé l'un de l'autre, sur deux côtés longitudinaux parallèles, caractérisé par le fait que la colonne (8) possède, en coupe transversale, un profil en forme de boîte fermée et que les rails de guidage sont réalisés sous la forme de renfoncements (17, 18), qui s'ouvrent vers l'extérieur dans deux parois parallèles de la colonne (8) et sont formés dans les parois elles-mêmes du profil en forme de boîte.

FIG 1

EP 0 245 584 B1

8

17

18

20

21

19

FIG 2